# EUROPEAN PATENT APPLICATION

(11) **EP 2 353 617 A2**
(43) Date of publication of application: **10.08.2011**
(21) Application number: 11150576.4
(22) Date of filing: 11.01.2011
(51) Int. Cl.: A61L 9/14, B65D 83/14

(54) **Method and Apparatus for Killing Pathogens**

(30) Priority: 12.01.2010 US 685904
(71) Applicant: Pathocept Corporation, Toronto, ON M2N 6K8 (CA)
(72) Inventor: Benson, Ronald, North York Ontario M2P 3B6 (CA); Gangbar, Dean Robert, Toronto Ontario M4M 1R9 (CA)
(74) Representative: Jackson, Jonathan Andrew

(57) **Abstract**

Delivery mechanisms for dispersing a pathogen disinfectant liquid into the air or onto a surface are disclosed. There is an apparatus including a container comprising a sunlight-resistant compartment containing a pathogen disinfectant liquid, a propellant, and an actuator coupled to a valve for opening and closing the valve, wherein opening of the valve causes the propellant to move at least a portion of the pathogen disinfectant liquid through an orifice in the container. Also disclosed are a system and method for dispersing a pathogen disinfectant liquid into the air in an indoor environment where a pathogen disinfectant liquid is dispersed into circulating air in a HVAC system to disinfect the air in the indoor environment. Further disclosed is an apparatus for dispersing a pathogen disinfectant liquid into the air from a cartridge through the use of an automotive lighter receptacle or USB port.

## Description

### FIELD OF THE INVENTION

The present invention relates, in general but not exclusively to pathogen disinfectants, and more particularly, to a system, method and apparatus for killing pathogens. This may be achieved through dispersing a pathogen disinfectant liquid.

### BACKGROUND OF THE INVENTION

Pathogens are biological agents, such as bacteria, viruses, fungi, spores and parasites, that cause disease or illness in a host, such as a human. Despite the use of medical treatments such as vaccination, antibiotics and fungicide to guard against infection by pathogens, pathogens continue to threaten human health. The influenza A (H1N1) virus is an example of a highly contagious and dangerous airborne pathogen. Other pathogens include pseudomonas aeroginosa, listeria, salmonella, staphylococcus aureus, e. coli, poliovirus, rhinovirus, human coronavirus, norovirus, tricophyton mentagrophytes, stachybotrys chartarum, bacillus subtilis and cyptosporidum oocysts.

Airborne pathogens, such as the H1N1 virus, tend to best survive in controlled indoor environments such as commercial buildings, residences and hospitals. These indoor environment typically include a Heating, Ventilating, and Air Conditioning system ("HVAC system") which circulates air in the indoor environment, and as such can widely disperse the airborne pathogens.

Various disinfectants have been used to kill pathogens, especially surface pathogens, including alcohols, aldehydes, oxidizing agents, phenolics, and quaternary ammonium compounds. For example, sodium hypochlorite (chlorine-based household bleach), an oxidizing agent, is very commonly used to disinfect drains, toilets, and other surfaces. In more dilute forms, it is also used in swimming pools and in drinking water.

However, most disinfectants are potentially harmful, and even toxic, to humans and animals. It would be desirable to eliminate airborne or surface pathogens in an indoor environment in a convenient and cost-effective manner with a product that is safe for use around humans and animals. Accordingly, there remains a need for improvements in the art.

### BRIEF SUMMARY OF THE INVENTION

According to one aspect, the present invention provides an apparatus for dispersing a pathogen disinfectant liquid comprising: a container comprising a compartment configured for containing the pathogen disinfectant liquid; a propellant; and an actuator coupled to a valve for opening and closing the valve, wherein opening of the valve causes the propellant to move at least a portion of the pathogen disinfectant liquid through an orifice in the container.

According to another aspect, the present invention provides a system for dispersing a pathogen disinfectant liquid into the air comprising: a supply of the pathogen disinfectant liquid; a disperser configured for dispersing the pathogen disinfectant liquid; and the disperser being coupled to a HVAC system for circulating air in the indoor environment, the disperser being configured for dispersing the pathogen disinfectant liquid into the circulating air.

According to another aspect, the present invention provides a method of killing airborne pathogens in an indoor environment comprising: supplying a pathogen disinfectant liquid; and dispersing the pathogen disinfectant liquid into circulating air in a HVAC system in the indoor environment.

According to another aspect, the present invention provides an apparatus for dispersing a pathogen disinfectant liquid into the air comprising: a cartridge containing a pathogen disinfectant liquid; and a portable disperser comprising a power adapter configured for coupling to a power source, the portable disperser configured to receive the cartridge and to disperse at least a portion of the pathogen disinfectant liquid from the cartridge.

According to another aspect, the present invention provides a method of manufacturing an apparatus for dispersing a pathogen disinfectant liquid comprising: inserting a compartment configured for containing the pathogen disinfectant liquid into a container; filling the container, except for the compartment, with a propellant; injecting the pathogen disinfectant liquid into the compartment; and attaching an actuator to the container, the actuator coupled to a valve for opening and closing the valve, wherein opening of the valve causes the propellant to move at least a portion of the pathogen disinfectant liquid through an orifice in the container.

Other aspects and features according to the present application will become apparent to those ordinarily skilled in the art upon review of the following description of embodiments of the invention in conjunction with the accompanying figures.

### BRIEF DESCRIPTION OF THE DRAWINGS

Reference will now be made to the accompanying drawings which show, by way of example, embodiments of the invention, and how they may be carried into effect, and in which:

FIG. 1 is a cross-sectional view of an apparatus according to an embodiment of the present invention;

FIG. 2 is a flow diagram showing a process to fill the apparatus of FIG. 1 with pathogen disinfectant liquid;

FIG. 3 is a schematic diagram of a system according to an embodiment of the present invention;

FIG. 4 is a flow diagram of a method for killing or neutralizing airborne pathogens according to an embodiment of the present invention; and

FIG. 5 is a cross-sectional view of an apparatus according to an embodiment of the present invention.

Like reference numerals indicate like or corresponding elements in the drawings.

### DETAILED DESCRIPTION OF THE EMBODIMENTS

Embodiments of the present invention are generally directed to a system, method and apparatus for killing or neutralizing pathogens through dispersing a pathogen disinfectant liquid.

According to embodiments of the invention, the pathogen disinfectant liquid dispersed may inactivate or kill a variety of airborne and surface pathogens (such as bacteria, fungi and viruses), thereby acting as a disinfectant and/or an odour neutralizer. According to an embodiment, the active ingredient of the pathogen disinfectant liquid is sodium hypochlorite, constituted of hyper-activated hypochlorous acid. According to an embodiment, salt (NaCl) may also be suspended in the hypochlorous solution. Hypochlorous acid is effective at killing pathogens by inactivating proteins in bacteria that are essential for bacterial growth. However, it is not very stable and readily decomposes when exposed to oxygen in the air and especially when exposed to sunlight. According to an embodiment, the pathogen disinfectant liquid may be formulated to decompose to water (H₂O) (and salt (NaCl) if salt is suspended in the hypochlorous acid solution) and as such may be safely used around humans, animals, food and plants.

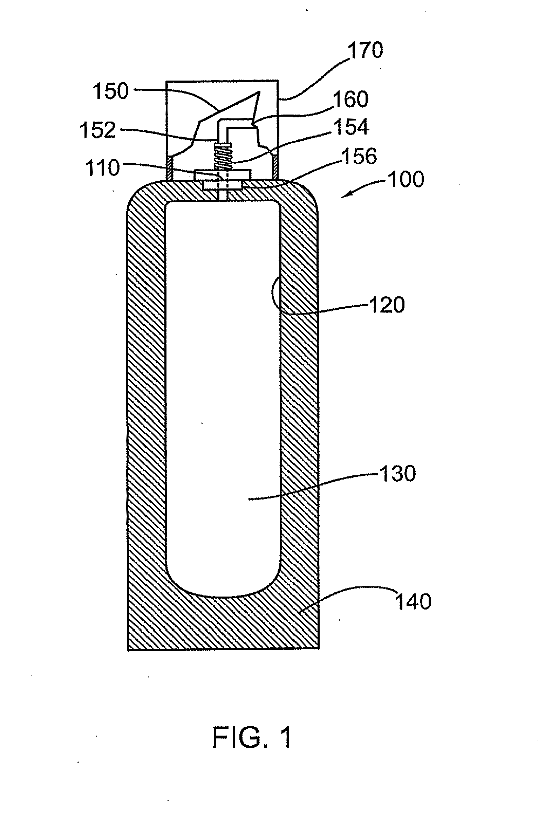

According to an embodiment as shown in FIG. 1, an apparatus comprises a container 100 including a valve 110, a compartment such as flexible bag 120, a pathogen disinfectant liquid 130, a propellant such as compressed gas 140, an actuator 150, an orifice such as nozzle 160, and a cap 170. According to an embodiment, the container 100 comprises an aerosol can made of aluminum or tin plate, or any other material suitable to withstand the pressure of the propellant without deforming. According to another aspect, the material may be shaped as desired. According to an embodiment, the container 100 comprises sunlight-resistant material, coating or skin so as to shield the pathogen disinfectant liquid 130 from sunlight which may cause it to decompose more rapidly. The cap 170 comprises a standard actuator overcap which detachably attaches to the container 100 to prevent accidental release of the pathogen disinfectant liquid 130 from the container 100.

The valve 110 may be pre-coupled to the flexible bag 120, according to an embodiment. The valve 110 may be either a male or female valve. The actuator 150 comprises a standard actuator that may be coupled to the valve 110 such that the valve 110 may be opened by depressing the actuator 150. According to an embodiment, actuator 150 includes a piston 152 and a spring 154 such that the spring 154 opposes the piston 152 to urge the piston 152 to its rest position after being depressed. According to an embodiment, a gasket 156 may also be employed to make the joint between the container 100 and actuator 150 fluid tight. According to an embodiment, the flexible bag 120 comprises a multilayer flexible bag and may be sized to fill a portion of the interior volume of the container 100. According to an embodiment, the flexible bag 120 comprises a high-density polyethylene inside liner layer, an oriented polyamide (OPA) nylon layer, an aluminum layer and a polyethylene terephthalate (PET) layer. According to a further embodiment, a polypropelyne inside liner layer may be used. As shown in FIG. 1, the flexible bag 120 may hold the pathogen disinfectant liquid 130. The pathogen disinfectant liquid 130 may be held in the flexible bag 120 and is separated from the compressed gas 140, i.e. the propellant. The bag and valve design described herein protects the pathogen disinfectant liquid 130 from contamination from external agents.

According to an embodiment, one of the layers of the flexible bag 120 comprises a sunlight-resistant material so as to shield the pathogen disinfectant liquid 130 from sunlight which may cause it to decompose more rapidly. It will be appreciated that this may increase the shelf life of the pathogen disinfectant liquid 130. According to an embodiment, the shelf life of the pathogen disinfectant liquid 130 in the container 100 may be at least 6 months.

According to an embodiment, the pathogen disinfectant liquid 130 comprises hyper-activated hypochlorous acid within a range of about 0.01% to 0.06% by concentration, which according to an embodiment may be measured according to the diethyl-p-phenylenediamine (DPD) method of measuring chlorine residual in water. According to an embodiment, the compressed gas 140 or propellant comprises compressed nitrogen. Nitrogen, unlike some other propellants, it is not a volatile organic compound (i.e. a greenhouse gas) and may be preferred for environmental reasons. According to a further embodiment, the compressed gas 140 comprises compressed air. The compressed gas 140 acts on the flexible bag 120 and when the valve 110 opens, the pathogen disinfectant liquid 130 may be pushed out through the nozzle 160 without mixing with the compressed gas 140 and without allowing external agents to enter the container 100 through the nozzle 160. The pathogen disinfectant liquid 130 exits the container 100 as a nebulisation, i.e. a fine spray with small airborne liquid droplets. The shape of the nozzle 160 determines the shape of the spray cone. For example, a wide spraying cone allows for a finer spray, which increases the effectiveness of the spray.

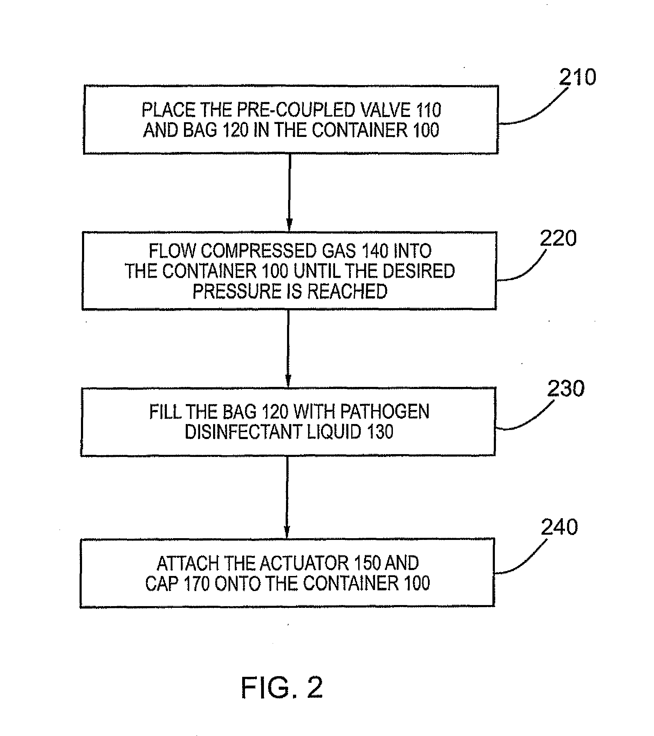

Embodiments of the apparatus may be filled with the pathogen disinfectant solution 130 as shown in FIG. 2 as follows. Step 210 comprises placing the pre-coupled valve 110 and flexible bag 120 in the container 100. Step 220 comprises flowing or filling the compressed gas 140 into the container 100 until the desired pressure is reached. According to an embodiment, compressed nitrogen may be added to the container 100 until a pressure of about 3 to 4 bar at 20 degrees Celsius is reached. Step 230 comprises filling the flexible bag 120 with the pathogen disinfectant liquid 130 by injecting it under pressure into the flexible bag 120. According to an embodiment, a pressure of 8 to 10 bar at 20 degrees Celsius is reached after filling the flexible bag 120 with the pathogen disinfectant liquid 130. According to an embodiment, the container 100 may now be weighed to determine whether the desired amount of pathogen disinfectant liquid 130 has been added to the flexible bag 120. As the compressed gas should weigh very little, and the weight of the container 100, bag 120 and valve 110 may be measured in advance so as to be known, the weight of the pathogen disinfectant liquid 130 is easy to calculate. Step 240 comprises attaching the actuator 150 and the cap 170 to the container 100.

In operation, the container 100 may be held up and sprayed in all directions to mist the air thoroughly to inactivate or kill airborne pathogens. According to another aspect, to inactivate or kill pathogens on surfaces such as workstations or furniture, the container 100 may be held approximately six to twelve inches from a pre-cleaned surface and the pathogen disinfectant liquid may be sprayed until mist covers the entire area. Then, the sprayed area may be left moist for approximately 30 to 60 seconds before wiping.

When compressed gas 140 is used as the propellant, it may be prudent to caution the user not to puncture or incinerate the container 100 or store it at high temperatures, such as over approximately 120 degrees Fahrenheit, as the container 100 may explode.

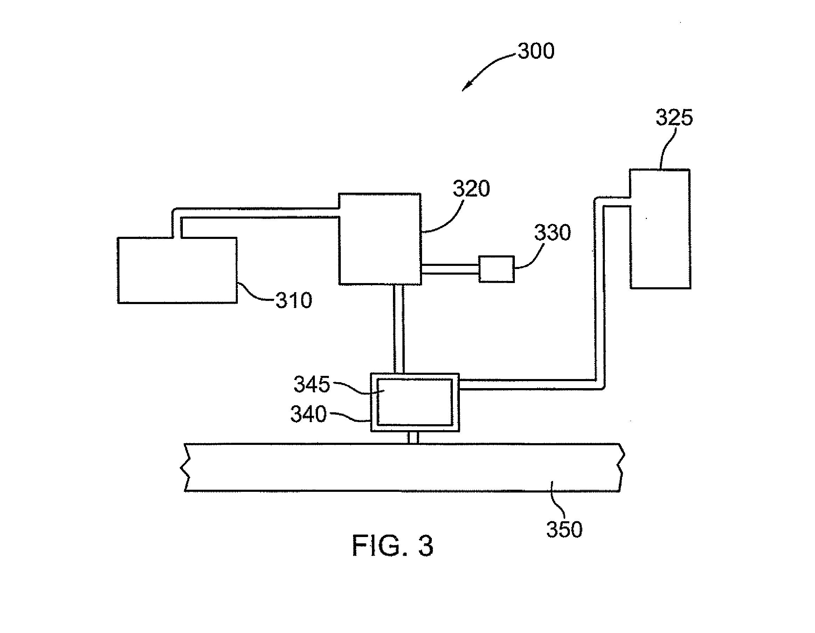

A system according to an embodiment is shown in FIG. 3 and indicated generally by reference 300. The system 300 comprises a supply of salt water 310 which flows into a pathogen disinfectant liquid generator such as electrolyzer 320 to generate a pathogen disinfectant liquid 130 which in the embodiment shown comprises a hypochlorous acid solution. The resultant pathogen disinfectant liquid 130 may be dispersed by a disperser 340 such as a cold air diffuser into a portion of a HVAC system 350, such as an air duct, to disinfect circulating air as it circulates through an indoor environment.

According to a further embodiment of the system, the pathogen disinfectant liquid 130 may be supplied by a supply of pathogen disinfectant liquid 325, such as containers of ready to use pathogen disinfectant liquid 130, thereby obviating the need to have an electrolyzer 320 as a component of the delivery system.

The electrolyzer 320 may be supplied or powered with direct electric energy, via an electric power source such as a power outlet 330 or a battery. The electrolyzer 320 may be configured to perform electrolysis, which is a method of using electric current to drive an otherwise non-spontaneous chemical reaction. According to an embodiment, the electrolyzer 320 includes two electrodes which may be in the form of two solid rods or plates made from an inert metal such as platinum or stainless steel. A supply of salt water solution 310 may be fed to the electrolyzer 320 such that the electrodes are immersed in the salt water solution 310. Electric current may then be used to drive the chemical reaction which generates the pathogen disinfectant liquid 130. According to an embodiment of the system, the pathogen disinfectant liquid 130 comprises a solution of water and hypochlorous acid.

The supply of salt water solution 310 entering the electrolyzer 320 may be premixed, or mixed within the indoor environment where the embodiment of the system 300 may be deployed. According to an embodiment, the salt water solution may be prepared in two stages. The first stage involves preparing a mixture of a salt water solution where the salt may be about 16-26% of the mixture by weight. The second stage is to add water to the salt water solution until the salt in the salt water solution may be about 0.5% of the mixture by weight. Water softeners may be used to remove heavy metals and sedimentation from the water prior to mixing if desired. According to an embodiment, the resultant pathogen disinfectant liquid 130 after electrolysis comprises a hypochlorous acid solution with about 0.01 to 0.06% hypochlorous acid by concentration. Byproducts of the electrolysis process are separately disposed of from the electrolyzer 320, resulting in only the pathogen disinfectant liquid 130 being provided to the disperser 340, e.g. a cold air diffuser.

The disperser 340 may be configured to use hot, cold or piezoelectric technology to disperse or aerosolize the pathogen disinfectant liquid 130. The spray cone and spray power of the disperser 340 should be configured to suit the cross-sectional area of the portion of the HVAC system 350, such as an air duct, where the pathogen disinfectant liquid 130 may be dispersed. Depending on the configuration of the disperser 340, a greater spray cone and spray power may be needed where the cross-sectional area of the portion of the HVAC system 350 to which the disperser 340 disperses the pathogen disinfectant liquid 130 is large. According to an embodiment of the system 300, the disperser 340 comprises a sunlight-resistant enclosure or skin. According to a further embodiment, the electrolyzer 320 may comprise a sunlight-resistant enclosure or skin.

A cold air diffuser is a type of disperser 340 which may produce a micro fine mist that may be visible, precise and efficient. This mist may suspend the pathogen disinfectant liquid 130 for several hours. The cold air diffuser 340 may disperse the pathogen disinfectant liquid 130 without heating or burning it which may serve to preserve the integrity of the pathogen disinfectant liquid 130.

According to an embodiment of the system 300, the cold air diffuser 340 comprises a chamber 345 which receives and contains the pathogen disinfectant liquid 130. The chamber 345 of the cold air diffuser 340 receives the pathogen disinfectant liquid 130 from the electrolyzer 320 or from a separate container or source. According to an embodiment, the conduit between the cold air diffuser 340 and the electrolyzer 320 may be permanently sealed with a liquid solder or other suitable sealant to prevent air from mixing with the pathogen disinfectant liquid 130 and any of the pathogen disinfectant liquid 130 from escaping the chamber 345 (other than by dispersion from the cold air diffuser 340 according to normal operation). This helps protect the pathogen disinfectant liquid 130 from contamination during the delivery process.

According to an embodiment of the system 300, the disperser 340 may be configured for dispersing the pathogen disinfectant 130 liquid within approximately 30 days of receiving the pathogen disinfectant liquid 130.

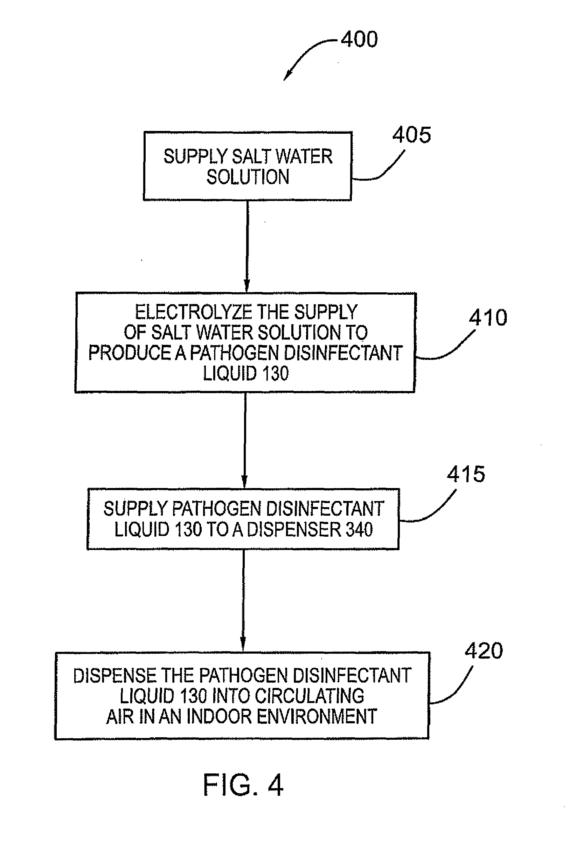

A method for killing or neutralizing airborne pathogens according to an embodiment of the invention is shown in FIG. 4 and indicated generally by reference 400. Step 415 comprises supplying the pathogen disinfectant liquid 130 to a disperser 340 (FIG. 3). Step 420 comprises dispersing the pathogen disinfectant liquid 130 into circulating air in a portion of a HVAC system 350 (FIG. 3), such as an air duct, in an indoor environment. According to a further embodiment, step 410 in the method or process 400 comprises electrolyzing a supply of salt water solution 310 (FIG. 3) to produce the pathogen disinfectant liquid 130. According to a yet further embodiment, the method 400 includes step 405 which comprises supplying salt water solution 310.

According to an embodiment of the method 400, the pathogen disinfectant liquid 130 comprises a solution of water and hypochlorous acid. According to a further embodiment of the method 400, the pathogen disinfectant liquid 130 may be dispersed into circulating air in the HVAC system 350 (FIG. 3) within approximately 30 days of being supplied to the disperser 340 (FIG. 3).

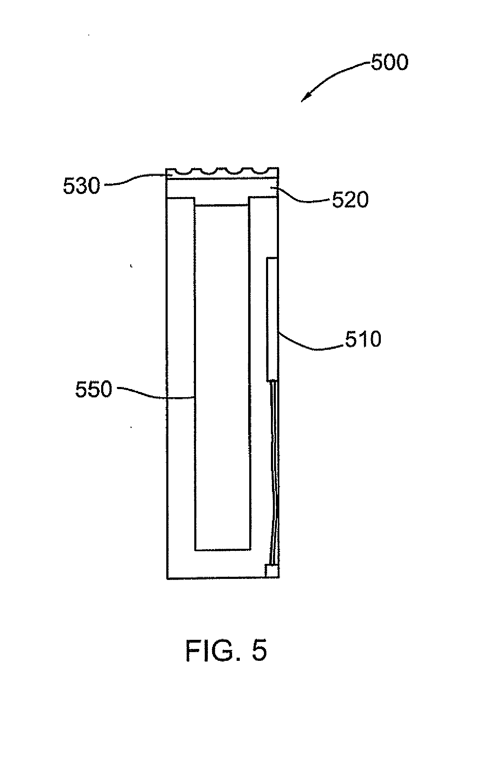

According to an embodiment of an apparatus as shown in FIG. 5, a portable disperser 500 comprises a power adaptor 510, control circuitry 520 and one or more orifices 530 and may be configured to receive a cartridge 550 containing a pathogen disinfectant liquid 130. The portable disperser 500 may be configured to disperse at least a portion of the pathogen disinfectant liquid 130 from the cartridge 550 through the one or more orifices 530 of the portable disperser 500. According to an embodiment, the cartridge 550 may be a replaceable cartridge. According to a further embodiment, the portable disperser 500 disperses a portion of the pathogen disinfectant liquid 130 from the cartridge 550 at periodic intervals. According to another embodiment, the power adaptor 510 may be coupled to an automotive lighter receptacle to power the portable disperser 500. According to yet another embodiment, the power adaptor 510 may be coupled to a USB port to power the portable disperser 500. According to a further embodiment, at least one of the pathogen disinfectant liquid cartridge 550 and the portable disperser 500 comprises a sunlight-resistant material.

As set out below, the pathogen disinfectant liquid 130 according to an embodiment of the invention has been shown to be effective at inactivating or killing a variety of airborne and surface pathogens (such as bacteria, fungi and viruses). In one study, an embodiment of the pathogen disinfectant liquid 130 provided the following reductions in harmful microorganisms:

**Table 1: Effectiveness of Pathogen Disinfectant Liquid 130 at Killing Pathogens**

| | **Microorganism** | **% Reduction** |
|---|---|---|
| Bacteria | Pseudomonas aeroginosa | 100% |
| | Listeria | 100% |
| | Salmonella | 100% |
| | Staphylococcus aureus | 100% |
| | E. coli | 100% |
| Virus | Influenza A (H1N1) | > 99.997% |
| | Poliovirus | > 99.9% |
| | Rhinovirus | > 99.997% |
| | Human Coronavirus | > 99.997% |
| | Norovirus | > 99.99% |
| Fungus | Tricophyton mentagrophytes | 100% |
| | Stachybotrys chartarum | 100% |
| Spores | Bacillus subtilis | > 99.98% |
| Parasites | Cyptosporidum oocysts | > 99% |

In a further study and application, the pathogen disinfectant liquid 130 was aerosolized and Petri dishes containing a dry microbial film were placed face down approximately one inch above the discharge port of the aerosolizer. Each of the inoculated Petri dishes was exposed for 105 seconds to the emitted aerosols from the aerosolizer. Additionally, unexposed dishes were used as controls to determine the level of microbial load contamination prior to disinfectant exposure. Fifteen milliters of neutralizing buffer was added to each dish immediately following exposure and to unexposed control plates. Next, the contents of the dishes were transferred to sterile conical bottom polypropylene centrifuge tubes. The tubes were then agitated briefly. The number of viable bacteria in each of the tubes was enumerated by spread plating onto plate count agar and incubating at 37 Celsius for 24 hours. Bacteriophage was enumerated by a single layer agar overlay procedure using the *E. coli* C3000 host as per EPA 1602. The results of the enumerations are shown in Table 2 below:

**Table 2: The Reduction of Methicillin Resistant Staphylococcus aureus (MRSA) and Bacteriophage MS2 Following Exposure to Aerosolized Pathogen Killing Liquid 130**

| **Microorganism** | **Plaque Forming Units (PFU)/ml of Bacteriophage MS2 and Colony Forming Units (CFU)/ml of MRSA and Resulting Percent Reduction Following Exposure*** | | |
|---|---|---|---|
| | **Initial (untreated)** | **Treated 2 Minutes** | **Average Percent Reduction** |
| **MS2** | 2.1 x 10^4 | 1.8 x 10^3 | 86.8% |
| | 1.1 x 10^4 | 2.4 x 10^3 | |
| | | 3.6 x 10^3 | |
| | 1.4 x 10^4 | 2.1 x 10^3 | |
| | | 3.8 x 10^3 | |
| **MRSA** | 1.6 x 10^4 | 1.8 x 10^2 | 97.4% |
| | | 3.3 x 10^2 | |
| | 2.6 x 10^4 | 5.1 x 10^2 | |
| | | 7.3 x 10^2 | |
| | 1.3 x 10^4 | 6.9 x 10^2 | |
| | | | |

As set out above, the pathogen disinfectant liquid 130 has been shown to be effective to inactivate or kill bacteria, fungi and viruses with a safe and environmentally friendly formula. The apparatus, system and/or method according to embodiments of the present invention may therefore be suitable for use around humans, animals, foods and plants in such places as hospitals, clinics and nursing homes, government offices and public buildings, medical and dental offices, veterinary clinics, hotels, ships and airplanes, restaurants and food courts, schools and daycares, athletic facilities and homes.

The present invention may be embodied in other specific forms without departing from the spirit or essential characteristics thereof. Certain adaptations and modifications of the invention will be obvious to those skilled in the art. Therefore, the presently discussed embodiments are considered to be illustrative and not restrictive, the scope of the invention being indicated by the appended claims rather than the foregoing description, and all changes which come within the meaning and range of equivalency of the claims are therefore intended to be embraced therein.

In a system method, apparatus or method of manufacture described above, the pathogen disinfectant liquid comprises a solution of water and hypochlorous acid. The disperser may comprise a cold air diffuser. The disperser may be coupled to an air duct of the HVAC system. In a method described above, the step of supplying the salt water solution. In the apparatus, the power source may comprise an automotive lighter receptacle. In the apparatus the power source may comprise a USB port. In the method of manufacturing the apparatus, the method may have the propellant as a compressed gas which may be nitrogen. In the method of manufacture, at least one of the container, and compartment comprises a sunlight-resistant material. The orifice may comprise a nozzle for outputting the pathogen disinfectant liquid as an aerosol spray and the compartment may comprise a flexible material.

## Claims

1. An apparatus for dispersing a pathogen disinfectant liquid comprising:
a container comprising
a compartment configured for containing the pathogen disinfectant liquid;
a propellant; and
an actuator coupled to a valve for opening and closing the valve, wherein opening of the valve causes the propellant to move at least a portion of the pathogen disinfectant liquid through an orifice in the container.

2. The apparatus of claim 1, wherein at least one of the container and the compartment comprises a sunlight-resistant material.

3. The apparatus of claim 1, wherein the orifice comprises a nozzle for outputting the pathogen disinfectant liquid as an aerosol spray.

4. The apparatus of claim 1, wherein the propellant comprises a compressed gas.

5. The apparatus of claim 1, wherein the pathogen disinfectant liquid comprises a solution of water and hypochlorous acid.

6. The apparatus of claim 1, wherein the compartment comprises a flexible material.

7. A system for dispersing a pathogen disinfectant liquid into the air comprising:
a supply of the pathogen disinfectant liquid;
a disperser configured for dispersing the pathogen disinfectant liquid; and
the disperser being coupled to a HVAC system for circulating air in the indoor environment, the disperser being configured for dispersing the pathogen disinfectant liquid into the circulating air.

8. The system of claim 7, further comprising an electrolyzer configured for generating the supply of the pathogen disinfectant liquid from a supply of salt water solution.

9. A method of killing airborne pathogens in an indoor environment comprising:
supplying a pathogen disinfectant liquid; and
dispersing the pathogen disinfectant liquid into circulating air in a HVAC system in the indoor environment.

10. The method of claim 9, further comprising electrolyzing a salt water solution to produce the pathogen disinfectant liquid.

11. An apparatus for dispersing a pathogen disinfectant liquid into the air comprising:
a cartridge containing a pathogen disinfectant liquid; and
a portable disperser comprising a power adapter configured for coupling to a power source, the portable disperser configured to receive the cartridge and to disperse at least a portion of the pathogen disinfectant liquid from the cartridge.

12. The apparatus of claim 11, wherein at least one of the cartridge and the portable disperser comprises a sunlight-resistant material.

13. A method of manufacturing an apparatus for dispersing a pathogen disinfectant liquid comprising:
inserting a compartment configured for containing the pathogen disinfectant liquid into a container;
filling the container, except for the compartment, with a propellant;
injecting the pathogen disinfectant liquid into the compartment; and
attaching an actuator to the container, the actuator coupled to a valve for opening and closing the valve, wherein opening of the valve causes the propellant to move at least a portion of the pathogen disinfectant liquid through an orifice in the container.

14. The method of claim 13, wherein the propellant is compressed nitrogen and wherein the container, except for the compartment, is filled with the propellant to a pressure of about 3 bar to about 4 bar.

15. The method of claim 13, wherein the pathogen disinfectant liquid comprises a solution of water and hypochlorous acid and wherein the compartment is injected with the pathogen disinfectant liquid until a pressure of about 8 bar to about 10 bar is reached.
